Europäisches Patentamt

European Patent Office

Office européen des brevets

(11) Veröffentlichungsnummer: **0 330 760 B1**

# EUROPÄISCHE PATENTSCHRIFT

(45) Veröffentlichungstag der Patentschrift: **04.05.94**

(51) Int. Cl.⁵: **G01N 33/24**

(21) Anmeldenummer: **88121661.8**

(22) Anmeldetag: **24.12.88**

Die Akte enthält technische Angaben, die nach dem Eingang der Anmeldung eingereicht wurden und die nicht in dieser Patentschrift enthalten sind.

(54) **Verfahren zur Ermittlung einer optimalen Düngemittelmenge.**

(30) Priorität: **04.03.88 DE 3807209**

(43) Veröffentlichungstag der Anmeldung:
**06.09.89 Patentblatt 89/36**

(45) Bekanntmachung des Hinweises auf die Patenterteilung:
**04.05.94 Patentblatt 94/18**

(84) Benannte Vertragsstaaten:
**AT BE CH FR GB IT LI LU NL**

(56) Entgegenhaltungen:
**DE-A- 814 527**
**GB-A- 505 381**

**PATENT ASSOCIETED LITERATURE, Laboratory Practice, Band 24, Nr. 7, Juli 1975; K.F. BAKER, Seiten 469-471&NUM;**

**K. SHRIVER, "Optimization of Fertilizer Recommendations via EDP in the Danish Agricultural Advisory Service", FAO Seminar, Jänner 1982, Genf (CH);**

(73) Patentinhaber: **KALI UND SALZ AKTIENGE-SELLSCHAFT**
**Postfach 10 20 29**
**D-34111 Kassel(DE)**

(72) Erfinder: **Andres, Ernst, Dr.**
**Lambertweg, 46**
**D-3500 Kassel(DE)**
Erfinder: **Orlovius, Kristian, Dr.**
**Balhorner Weg 1**
**D-3501 Emstal-Sand(DE)**

EP 0 330 760 B1

**Beschreibung**

Die Erfindung bezieht sich auf ein Verfahren zur Ermittlung der optimalen Kali-Düngemittelmenge unter Berücksichtigung des im Boden vorhandenen Nährstoffvorrates.

Es ist bekannt, die Erträge von Kulturpflanzen, wie z.B. Weizen, Gerste usw. durch Düngung, insbesondere mit Mineralstoffen, wie z.B. Stickstoff, Magnesium, Kali, Phosphat zu erhöhen. Erkannt wurde auch der Zusammenhang zwischen Standort der Pflanze und der Höhe der zuzugebenden Düngemittelmenge, wobei auch bekannt ist, daß es auf die Kaliverfügbarkeit ankommt, die wiederum von den verschiedensten Einflußgrößen abhängt. Dabei ist auch bekannt die Feststellung der Düngemittelmenge durch Feldversuche zu ermitteln.

Im einzelnen ist man dabei so vorgegangen, daß man die Ergebnisse von Feldversuchen auf unterschiedlichen Standorten zusammengefaßt und Mittelwerte für die auszubringende Düngemittelmenge gebildet hat.

Das hat aber den Nachteil, daß die vorgeschlagenen Düngemittelmengen nur in den seltensten Fällen für einen bestimmten Standort optimal waren; bei den meisten anderen Standorten waren die vorgeschlagenen Düngemittelmengen entweder zu groß oder zu klein.

Zu kleine Düngemittelmengen mindern naturgemäß den Ertrag; zu große Düngemittelmengen stellen hingegen eine Umweltbelastung dar, ganz abgesehen von den wirtschaftlichen Nachteilen. Darüber hinaus vermindert sich der Ertrag nicht nur bei zu geringen Düngemittelmengen, sondern auch bei überhöhten Düngemittelgaben.

Der Erfindung liegt daher die Aufgabe zugrunde, ein Verfahren zur Ermittlung der optimalen Kali-Düngemittelmenge unter Berücksichtigung des im Boden vorhandenen Nährstoffvorrates zu schaffen, das auf den Standort bezogen optimal ist.

Das wird erfindungsgemäß

erstens durch Messung der für das Pflanzenwachstum maßgeblichen Standortfaktoren, und

zweitens durch Messung der nach erfolgter Düngung, unter Berücksichtigung der ermittelten Standortfaktoren, gewonnenen Erträge, und

drittens durch Ermittlung von Flächen bzw. Böden mit gleicher Standortfaktorenkombination in bestimmten Naturräumen erreicht.

Nach dem erfindungsgemäßen Verfahren werden Messungen zur Bestimmung folgender Standortfaktoren durchgefuhrt:

a) Bestimmung des Kalkgehaltes;

b) Bestimmung des Humusgehaltes;

c) Bestimmung des pH-Wertes;

d) Bestimmung der Korngrößenzusammensetzung in Sand, Schluff und Ton;

e) Bestimmung der Kationen-Austauschkapazität und der Ionenbelegung;

f) Bestimmung bestimmter Nährstofffraktionen, z.B. von Phosphaten, Kali und Magnesium;

g) Bestimmung der Kalifixierungskapazität;

h) Bestimmung der durchschnittliche Jahrestemperatur und durchschnittlice Niederschlagsmenge.

Bei diesem Verfahren werden demzufolge die optimalen Kali-Düngemittelmengen für bestimmte Pflanzenarten durch Feldversuche ermittelt, die unter bestimmten standortbezogenen Bedingungen durchgeführt werden, so daß bestimmten Standortbedingungen bestimmte Düngemittelmengen für bestimmte Pflanzenarten zugeordnet werden können.

Sind demzufolge in einer bestimmten Region die Standortbedingungen bekannt, so kann durch Vergleich der mit den durch Feldversuche unter gleichen Standortbedingungen gewonnenen Ergebnissen die erforderliche Kali-Düngemittelmenge, bezogen auf die jeweilige Pflanzenart, ermittelt werden.

Eine noch weitergehende Optimierung der Düngemittelmenge erfolgt dadurch, daß auch Bodentyp und geologisches Ausgangsmaterial bestimmt werden.

Nach einem besonderen Merkmal der Erfindung erfolgt die Messung zur Bestimmung der Nährstofffraktionen durch die Calium-Azetat-Lactat Methode.

Anhand eines Beispieles wird das Verfahren im folgenden für den Nährstoff Kali näher erläutert.

Der Düngungsversuch wurde auf einem Standort vorgenommen, der durch folgende Daten gekennzeichnet ist:

| | |
|---|---|
| Großlandschaft | westf.Tieflandsbucht; |
| Naturraum | Hellwegbörden; |
| Bodentyp | Parabraunerde; |
| geologisches Ausgangsmaterial | Löß (größer 2m) |

Folgende Standortfaktoren wurden ermittelt:

Tabelle 1:                                                    Parabraunerde aus Löß.

| | | | Tiefe (cm) | | | |
|---|---|---|---|---|---|---|
| | | 0 - 30 | 30 - 45 | 45 - 95 | 95 - 140 | 140 - 200 |
| | Horizont-bezeichnung | $A_p$ | $A_1$ | $B_t$ | $B_{v1}$ | $B_{v2}$ |
| a) | % $CaCO_3$ | Spuren | 0 | 0 | 0 | 0 |
| b) | % C | 1,70 | 0,70 | n. b. | n. b. | n. b. |
| | % N | 0,14 | 0,06 | n. b. | n. b. | n. b. |
| c) | $pH_{CaCl_2}$ | 6,8 | 6,5 | 6,2 | 6,1 | 6,2 |
| d) | Korngrößen-Anteile (%)   S | 6 | 6 | 5 | 9 | 6 |
| | U | 81 | 79 | 68 | 72 | 75 |
| | T | 13 | 15 | 27 | 19 | 18 |
| e) | $KAK_{pot.}$ (meq./100 g B.) | 12,3 | 9,9 | 15,1 | 10,7 | 10,2 |
| | Ionenbelegung meq/100 g B.   H | 3,0 | 3,0 | 5,0 | 3,0 | 3,0 |
| | Ca | 9,0 | 7,1 | 10,8 | 7,6 | 7,9 |
| | Mg | 0,9 | 0,6 | 0,6 | 0,4 | 0,4 |
| | K | 0,2 | 0,1 | 0,1 | 0,1 | 0,1 |
| | Na | 0,1 | 0,1 | 0,1 | 0,1 | 0,1 |
| f) | BU-Ergebnis mg/100 g B.   $P_2O_{5CAL}$ | 19 | 6 | 3 | 9 | 9 |
| | $K_2O_{CAL}$ | 9 | 5 | 5 | 5 | 4 |
| | $Mg_{CaCl_2}$ | 12 | 10 | 10 | 9 | 9 |
| g) | Kalifixierung (naß) (mg K/100 g B.) | 29 | 27 | 35 | 32 | 35 |

h) durchschnittl. Jahresniederschlag     731 mm
Jahresdurchschnittstemperatur          9° C

Unter Punkt f) ist hierbei das Ergebnis der Calzium-Azetat-Lactat Methode (Phosphat, Kali) und der Calzium-Chlorid-Methode (Magnesium) festgehalten.

In Abhängigkeit von der Kalidüngemittelmenge konnten folgende Erträge ermittelt werden:

Tabelle 2:          Parabraunerde aus Löß

BU-Ergebnis vor Anlage (mg/100 g Boden):

pH: 6,2      $P_2O_{5CAL}$ : 27      $K_2O_{CAL}$ : 26      $Mg_{CaCl_2}$ : 10

Laufzeit des Versuches: 1979-86      Fruchtfolge: ZR-WW-WG

| Ø Kali-Düngung | Relativerträge | | mg $K_2O_{CAL}$/100 g B. |
| (kg $K_2O$/ha·a) | Getreide | Zuckerrüben | Ø 1984-86 |
| --- | --- | --- | --- |
| 0 | 100* | 100** | 22 |
| 150 | 105 | 115 | 25 |
| 300 | 104 | 117 | 36 |

\* ≙ 6,92 t Korn/ha          \*\* ≙ 6,84 t Zucker/ha

Diese durch Feldversuche gewonnenen pflanzen-und Standortspezifischen optimalen Düngemengen werden auf Flächen bzw. Böden übertragen, die die gleichen Standortfaktoran bzw. Standortfaktorenkombinationen aufweisen, wie sie bei den Feldversuchen vorgelegen haben.

Es ist deutlich erkennbar, daß das Ertragsoptimum bei einer durchschnittlichen Kalidüngung von 150 kg/ha und Jahr liegt. Als Belag dafür, daß diese Düngung in bezug auf die Anreicherung des Bodens mit Kali optimal ist, dient die Tatsache, daß der Kaligehalt des Bodens während der Laufzeit des Versuches (1979 bis 1986) konstant blieb (Tabelle 2: hier ist in Zeile 3 der Kaligehalt des Bodens vor Anlage des Versuches und in der rechten Spalte der Gehalt an Kali pro 100 g Boden im Jahresdurchschnitt der Jahre 1984 bis 1986 angegeben).

Die Ermittlung von Flächen bzw. Böden mit gleichen Standortfaktoren geschieht durch die Kombination geographischer, geologischer, bodenkundlicher und klimatischer Informationen; mit Hilfe dieser Informationen lassen sich mehr oder weniger heterogene Bodenformengesellschaften, auch Pedochore genannt, abgrenzen, deren Pedotope (unter Pedotop vesteht man die kleinste Bodeneinheit) sich hinsichtlich ihrer Eigenschaften in bezug auf Nährstoffdynamik und Kalidüngereaktion ähnlich sind.

Die Informationen erhält man aus sog. Bodenkarten und geologischen Karten, sowie aus Karten, die die mittlere jährliche Niederschlagshöhe und das Jahresmittel der Lufttemperatur ausweisen.

Die Bodenkarten geben Aufschluß über Bodentyp, Bodenart und das vorherrschende Ausgangsgestein; die geologische Karte ergänzt die Aussagefähigkeit der Bodenkarten; denn nur der Vergleich der Informationen aus der geologischen Karte mit der Bodenkarte ermöglicht eine Abschätzung der Mächtigkeit der bodenbildenden Sedimentdecke bzw. der Wahrscheinlichkeit des Auftretens geschichteter Bodenprofile.

Schließlich ist ein weiteres Kriterium zur Abgrenzung die Untergliederung in die naturräumlichen Haupteinheiten oder Naturräume, als "naturgemäß abgegrenzte landschaftliche Einheiten", die "trotz aller Vielgestaltigkeit von Lage, Klima und Boden .... in sich verwandte Grundzüge der Standorteinheiten aufweisen und sich im ganzen von den benachbarten Gebieten deutlich abheben". Der Naturraum stellt also ein regionales Ordnungskriterium dar, mit dessen Hilfe die Variationsbreite der Standortfaktoren seine pedologische und klimatische Eingrenzung erfährt. Es lassen sich also durch Kombination der oben aufgeführten Kriterien Flächen bzw. Böden ausweisen, deren Standortfaktoren-Variation im Vergleich zur Bodenkarte deutlich reduziert ist.

Anhang zu den benutzten Abkürzungen:

Zu Tabelle 1:

Korngrößenanteile $\begin{matrix} S \\ U \\ T \end{matrix}$ = Korngrößenanteile in $\begin{matrix} Sand \\ Schluff \\ Ton \end{matrix}$

Horizontbezeichnung $A_P$ = Bearbeitungshorizont

$A_L$ = tonverarmter Oberbodenhorizont

$B_t$ = Unterbodenhorizont mit Tonanreicherung

$B_{V1}$, $B_{V2}$ = Unterbodenhorizonte, verwittert und verbraunt

$KAK_{Pot}$ = Kationen-Austauschkapazität

BU-Ergebnis mg/100 g B = Bodenuntersuchungsergebnis in mg/100 g Boden

meq = Milliäquivalent

| Zu Tabelle 2 | |
| --- | --- |
| BU-Ergebnis vor Anlage | = Bodenuntersuchungsergebnis vor Anlage des Versuches auf der Fläche (= Vor Beginn des Versuches) |
| ZR | = Zuckerrübe |
| WW | = Winterweizen |
| WG | = Wintergerste |

**Patentansprüche**

1. Verfahren zur Ermittlung der optimalen Kali-Düngemittelmenge, wobei folgende Standortfaktoren berücksichtigt werden:
   a) Korngrößenzusammensetzung in Sand, Ton und Schluff,
   b) Humusgehalt,
   c) pH-Wert,
   d) Nährstoff-Fraktionen, z.B. von Phosphat, Kali und Magnesium,
   e) Kationenaustauschkapazität und Ionenbelegung,
   f) Kali-Fixierungskapazität,
   g) Kalkgehalt,
   h) durchschnittliche Jahrestemperatur und durchschnittliche Niederschlagsmenge.
   und wobei nach erfolgter Kali-Düngung unter Berücksichtigung der ermittelten Standortfaktoren die gewonnenen Erträge gemessen und damit die optimale standort und pflanzenspezifische Kali-Düngemittelmenge ermittelt werden, dadurch gekennzeichnet, daß Flächen mit gleicher Standortfaktorenkombination in bestimmten Naturräumen ermittelt werden und die antimale Kali-Düngemittel menge auf diesen Flächen übertrogen werden.

**2.** Verfahren nach Ansprüchen 1 und 2, dadurch gekennzeichnet, daß die Messung zur Bestimmung der Nährstoff-Fraktion durch die Calcium-Azetat-Lactat Methode erfolgt.

**3.** Verfahren nach Anspruch 1, dadurch gekennzeichnet, daß die Flächen Böden sind.

**4.** Verfahren nach Ansprüchen 1 und 2, dadurch gekennzeichnet, daß zusätzlich zu den gemessenen Standortfaktoren Bodentyp und geologisches Ausgangsmaterial der Bodenbildung bestimmt werden.

**Claims**

**1.** Method of ascertaining the optimum dose of potash fertiliser, wherein the following local conditions are taken into account:
(a) grain size distribution in sand, clay and slate,
(b) humus content,
(c) pH,
(d) nutrient fraction, for example of phosphates, potash and magnesium,
(e) cation exchange capacity and ion content,
(f) potash fixing capacity,
(g) lime content,
(h) average annual temperature and rainfall,
and wherein the yields obtained are measured following fertilisation with potash taking said local conditions into account to determine the optimum dose of potash fertiliser for specific locations and crops, characterised in that regions having the same combination of local conditions in specific environments are discerned and said optimum dose of potash fertiliser is distributed over these regions.

**2.** Method according to claim 1, characterised in that determination of the nutrient fraction is carried out using the calcium-acetate-lactate method.

**3.** Method according to claim 1, characterised in that the regions are natural soils.

**4.** Method according to claims 1 and 2, characterised in that, in addition to the measured local conditions, soil type and geological source material of the soil are also specified.

**Revendications**

**1.** Procédé de détermination de la quantité optimale d'engrais potassique en prenant en compte les facteurs locaux suivants :
a) composition granulométrique du sable, de l'argile et du limon,
b) teneur en humus,
c) pH,
d) fractions d'élément fertilisant, par exemple de phosphate, de potassium et de magnésium,
e) capacité d'échange de cations et occupation par des ions,
f) capacité de fixation du potassium,
g) teneur en chaux,
h) température annuelle moyenne et précipitation moyenne,
et dans lequel on mesure, après avoir effectué la fumure potassique en tenant compte des facteurs locaux déterminés, les rendements obtenus et on détermine ainsi la quantité optimale,de fumure potassique, qui est propre au lieu et qui est spécifique aux plantes, caractérisé en ce qu'il consiste à trouver des surfaces ayant la même combinaison de facteurs locaux dans certains espaces naturels, et à transposer la quantité optimale d'engrais potassique à partir de ces surfaces.

**2.** Procédé suivant la revendication 1, caractérisé en ce qu'il consiste à effectuer la mesure destinée à la détermination de la fraction d'élément fertilisant par la méthode à l'acétate-lactate de calcium.

**3.** procédé suivant la revendication 1, caractérisé en ce que les surfaces sont des sols.

4. Procédé suivant la revendication 1 ou 2, caractérisé en ce qu'il consiste à déterminer, en plus des facteurs locaux mesurés, le type de sol et la matière géologique d'origine de formation du sol.